# EUROPEAN PATENT APPLICATION

(11) **EP 4 556 065 A1**
(43) Date of publication of application: **21.05.2025**
(21) Application number: 24212544.1
(22) Date of filing: 13.11.2024
(51) Int. Cl.: A61N 5/10

(54) **PARTICLE BEAM TREATMENT SYSTEM AND SUPPORT MEMBER FOR PARTICLE BEAM TREATMENT**

(30) Priority: 17.11.2023 JP 2023196030
(71) Applicant: Sumitomo Heavy Industries, Ltd., Tokyo 141-6025 (JP)
(72) Inventor: NAKAKITA, Masaru, Ehime, 792-8588, (JP)
(74) Representative: Louis Pöhlau Lohrentz

(57) **Abstract**

Provided are a particle beam treatment system (150) capable of performing accurate irradiation with a particle beam and a support member (80) for a particle beam treatment. A particle beam treatment system (150) includes a placement section (60) for placing a patient (50) to be irradiated with a particle beam. Therefore, an irradiation port (106) can irradiate a treatment site (55) of the patient (50) placed on the placement section (60) with the particle beam. With regard to this, the particle beam treatment system (150) irradiates the patient (50) with the particle beam from the irradiation port (106) in a state where the patient (50) placed on the placement section (60) is fixed in the lateral position. For example, as shown in Fig. 6A, when the patient (50) is in the lateral position, interference between the collimator (20) and the vicinity of a shoulder can be suppressed. Therefore, a distance (L1) between an emission port (20b) and the treatment site (55) can be reduced. In this way, by placing the patient (50) in the lateral position according to a position of the treatment site (55) of the patient (50), the treatment site (55) can be disposed near the emission port (20b) of the irradiation port (106). Furthermore, by fixing a posture of the lateral position of the patient in a state where the treatment site (55) is disposed near the emission port (20b), it is possible to suppress movement of the treatment site (55) during a treatment.

## Description

### BACKGROUND OF THE INVENTION

### Field of the Invention

The present invention relates to a particle beam treatment system and a support member for a particle beam treatment.

### Description of Related Art

A technology for performing a treatment by irradiating a patient with a particle beam is known (for example, Japanese Unexamined Patent Publication No. 2016-83351). Japanese Unexamined Patent Publication No. 2016-83351 describes a boron neutron capture therapy (BNCT) using a boron compound as a neutron capture therapy for killing a cancer cell by irradiating the cancer cell with a neutron ray. In the boron neutron capture therapy, boron that has been incorporated into a cancer cell in advance is irradiated with a neutron ray, resulting in scattering of heavy charged particles that selectively destroy the cancer cell.

In Japanese Unexamined Patent Publication No. 2016-83351, a treatment is performed by disposing a patient in front of an irradiation port for the neutron ray in a state where the patient is fixed to a treatment bed.

### SUMMARY OF THE INVENTION

Here, when the treatment is performed by irradiation with the particle beam, there are cases where it is preferable to place the patient in a state of a lateral position depending on a position of an affected part. However, since the lateral position is a burdensome posture for the patient, it is difficult to maintain the posture, and thus there is a problem in that it is difficult to accurately irradiate the affected part with the particle beam.

Therefore, an object of the present invention is to provide a particle beam treatment system capable of performing accurate irradiation with a particle beam and a support member for a particle beam treatment.

A particle beam treatment system according to the present invention includes: a placement section for placing a patient to be irradiated with a particle beam; and an irradiation section that irradiates the patient placed on the placement section with the particle beam, wherein the irradiation section irradiates the patient with the particle beam in a state where the patient placed on the placement section is fixed in a lateral position.

The particle beam treatment system according to the present invention includes the placement section for placing the patient to be irradiated with the particle beam. Therefore, the irradiation section can irradiate a treatment site of the patient placed on the placement section with the particle beam. With regard to this, the particle beam treatment system irradiates the patient with the particle beam from the irradiation section in a state where the patient placed on the placement section is fixed in the lateral position. By placing the patient in a lateral position according to the position of the treatment site of the patient, the treatment site can be disposed close to the irradiator. Furthermore, by fixing a posture of the lateral position of the patient in a state where the treatment site is disposed near the irradiation section, it is possible to suppress movement of the treatment site during a treatment. Accordingly, the treatment can be reliably performed by performing accurate irradiation with the particle beam.

The particle beam treatment system may further include: a support member that is capable of supporting the patient placed on the placement section from at least one side with respect to a torso of the patient. The patient can be easily fixed in the lateral position by using the support member. In addition, by supporting the patient from at least one side with respect to the torso, the movement of the patient is suppressed, and thus it is easy for the patient to maintain a comfortable posture.

The support member may be capable of supporting the patient placed on the placement section from both sides with respect to the torso. In this way, by supporting the patient from both sides with respect to the torso, the movement of the patient is suppressed, and thus it is easy for the patient to maintain a comfortable posture.

The support member may include a belt-shaped member that is windable around the patient. The patient can be fixed according to a physique or posture of the patient by using the belt-shaped member.

The support member may include a fulcrum that holds one end portion of the belt-shaped member, and the fulcrum may be disposed at a position higher than the placement section. For example, in a case where the fulcrums of both end portions of the belt-shaped member are located at positions lower than the placement section, the belt-shaped member applies a supporting force to press the patient toward a placement section side. Contrary to this, in a case where the fulcrum is disposed at a position higher than the placement section, it is possible to apply a supporting force in a direction other than the direction in which the placement section is pressed. Accordingly, it is easy to apply supporting forces to the patient from both sides with respect to the torso of the patient.

The support member may apply a supporting force to the torso from below and from both sides in a horizontal direction by winding the belt-shaped member around the torso of the patient placed on the placement section. In this case, by applying the supporting forces in multiple directions according to the physique and posture of the patient by using the belt-shaped member, the movement of the patient is suppressed, and thus it is easy for the patient to maintain a comfortable posture.

The support member may include a first member that supports the patient placed on the placement section from both sides with respect to the torso, and a second member. By using the support member unitized by the first member and the second member, it is possible to easily position and fix the patient.

The particle beam treatment system may further include: a pad member interposed in a space between the patient in a state of the lateral position and the placement section. Accordingly, a space between an arm and shoulder of the patient in the state of the lateral position, the torso, and the placement section can be filled with the pad member. Therefore, even in a case where the patient is in the lateral position with one arm and shoulder placed on a lower side, the movement of the patient is suppressed, and it is easy for the patient to maintain a comfortable posture.

The particle beam treatment system may further include a neutron capture therapy apparatus. In the case of the neutron capture therapy apparatus, since it is necessary to perform high-dose irradiation, it is highly necessary to bring the treatment site of the patient close to the irradiation section. Therefore, compared to a proton beam treatment or the like, the patient is more likely to be in a posture that is burdensome for the patient, such as a lateral position. Furthermore, since an irradiation time is long, a time for which the burden is applied to the patient increases. Therefore, by adopting a structure of the present invention for the neutron capture therapy apparatus, it is possible to suppress the burden on the patient and to perform accurate irradiation with a neutron ray.

A support member for a particle beam treatment according to the present invention is a support member for a particle beam treatment that supports a patient to be irradiated with a particle beam, in which the support member for a particle beam treatment is capable of fixing the patient placed on a placement section in a state of a lateral position.

The support member for a particle beam treatment according to the present invention can fix the patient placed on the placement section in the state of the lateral position. Therefore, by placing the patient in the lateral position using the support member according to the position of the treatment site of the patient, the treatment site can be disposed near the irradiation section. Furthermore, a state where the posture of the lateral position of the patient is fixed by the support member can be maintained in a state where the treatment site is disposed near the irradiation section, so that it is possible to suppress the movement of the treatment site during the treatment. Accordingly, the treatment can be reliably performed by performing accurate irradiation with the particle beam.

According to the present invention, it is possible to provide a particle beam treatment system capable of performing accurate irradiation with a particle beam and a support member for a particle beam treatment.

### BRIEF DESCRIPTION OF THE DRAWINGS

Fig. 1 is a schematic view showing a particle beam treatment system according to an embodiment of the present invention.
Fig. 2A is a schematic plan view of a treatment bed, and Fig. 2B is a schematic sectional view taken along line IIb-IIb shown in Fig. 2A.
Fig. 3 is a schematic plan view showing the treatment bed in a state of being loaded with a patient.
Fig. 4 is a schematic sectional view showing the treatment bed in the state of being loaded with the patient.
Fig. 5 is a schematic side view showing the treatment bed in the state of being loaded with the patient.
Figs. 6A and 6B are schematic views for describing a positional relationship between an emission port and a treatment site.
Figs. 7A to 7C are views showing a support member of a particle beam treatment system according to a modification example.
Fig. 8 is a view showing a support member of a particle beam treatment system according to a modification example.
Figs. 9A and 9B are views showing a support member of a particle beam treatment system according to a modification example.
Fig. 10 is a view illustrating a support member of a particle beam treatment system according to a modification example.

### DETAILED DESCRIPTION OF THE INVENTION

Hereinafter, a preferred embodiment of the present invention will be described in detail with reference to the accompanying drawings.

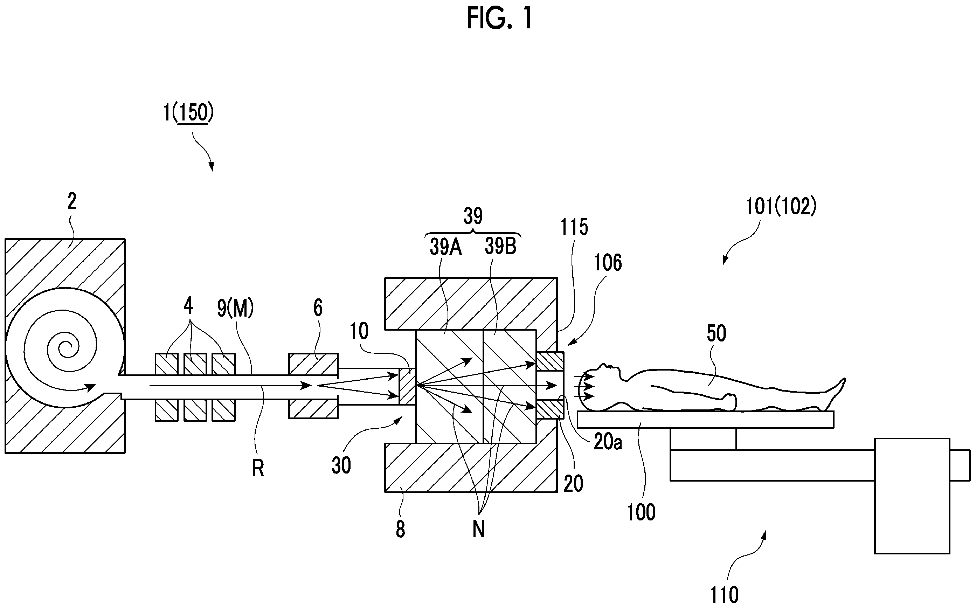

Fig. 1 is a schematic view showing a particle beam treatment system 150 according to an embodiment of the present invention. The particle beam treatment system 150 is a system that treats a patient using a particle beam. The particle beam treatment system 150 includes a neutron capture therapy apparatus 1. The neutron capture therapy apparatus 1 is an apparatus that performs a cancer treatment using a boron neutron capture therapy (BNCT). The neutron capture therapy apparatus 1 includes a treatment section 102, a treatment bed 100 (placement section), and a moving mechanism 110.

The treatment section 102 has an irradiation port 106 (irradiation section) that irradiates a patient 50 with a neutron ray N. The treatment section 102 is configured by a structure in which the irradiation port 106 or the moving mechanism 110 is disposed, and the like. The treatment section 102 is provided in a treatment room 101. The irradiation port 106 is provided on a vertical wall portion of the treatment room 101. The neutron ray N is emitted from the irradiation port 106 in a horizontal direction. The irradiation port 106 includes a collimator 20, which will be described later, and an irradiation section peripheral wall 115. The moving mechanism 110 is a mechanism capable of moving the treatment bed 100 on which the patient 50 is placed in the treatment section 102. The moving mechanism 110 is provided at a position in front of the irradiation port 106 in the treatment room 101.

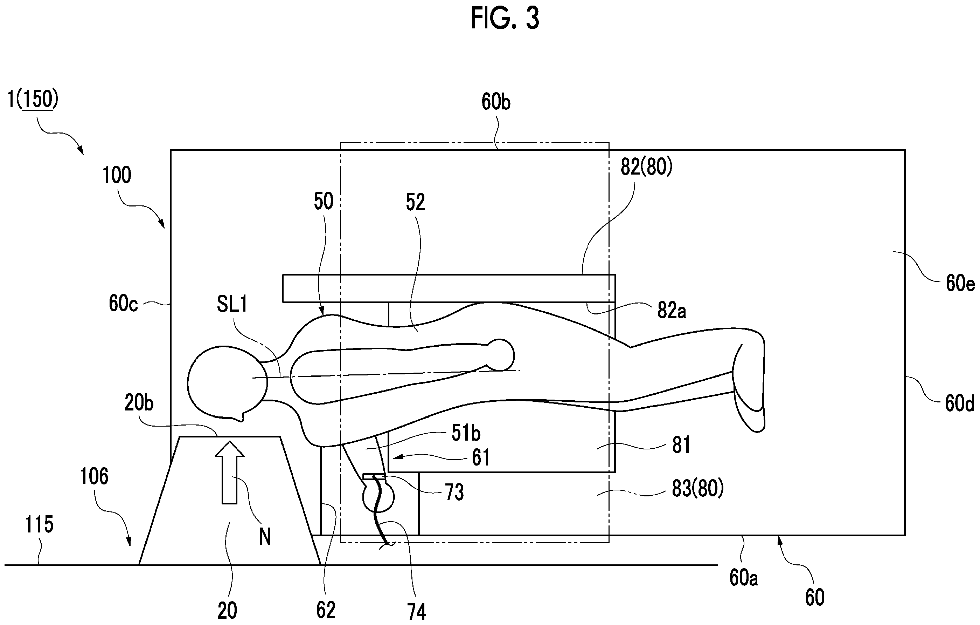

In the treatment section 102, for example, a tumor of the patient 50 to which boron (¹⁰B) is administered is irradiated with the neutron ray N. The irradiation port 106 irradiates the patient 50 placed on a placement section 60 (see Fig. 3) of the treatment bed 100 with the neutron ray N (particle beam).

The neutron capture therapy apparatus 1 includes an accelerator 2. The accelerator 2 accelerates particles to emit the particle beam R. For example, a cyclotron, a linear accelerator, or the like may be adopted as the accelerator 2.

The particle beam R emitted from the accelerator 2 passes through a transport path 9 called a beam duct, of which an inside is maintained in a vacuum and through which the beam can pass, and is transported to a target disposition section 30. The target disposition section 30 is a part where a target 10 is disposed, and has a mechanism that holds the target 10 in a posture for irradiation. The target disposition section 30 disposes the target 10 at a position facing an end portion (emission port) of the transport path 9. The particle beam R emitted from the accelerator 2 passes through the transport path 9 and advances toward the target 10 disposed at the end portion of the transport path 9. A plurality of electromagnets 4 (quadrupole electromagnets or the like) and a scanning electromagnet 6 are provided along the transport path 9. The plurality of electromagnets 4 perform, for example, beam axis adjustment of the particle beam R using the electromagnets.

The scanning electromagnet 6 scans the particle beam R and controls the irradiation of the target 10 with the particle beam R. The scanning electromagnet 6 controls an irradiation position of the particle beam R with respect to the target 10.

The neutron capture therapy apparatus 1 generates a neutron ray N by irradiating the target 10 with the particle beam R, and emits the neutron ray N toward the patient 50. The neutron capture therapy apparatus 1 includes the target 10, a shield member 8, a deceleration member 39, and the collimator 20.

The target 10 generates the neutron ray N by being irradiated with the particle beam R. The target 10 is a solid shape member formed of a material that generates the neutron ray N by being irradiated with the particle beam R. Specifically, the target 10 is formed of, for example, beryllium (Be), lithium (Li), tantalum (Ta), or tungsten (W), and has, for example, a solid shape in a disk shape having a diameter of 160 mm. The target 10 is not limited to the disk shape, and may have another shape.

The deceleration member 39 decelerates the neutron ray N generated by the target 10 (reduces an energy of the neutron ray N). The deceleration member 39 may have a laminated structure including a layer 39A that mainly decelerates fast neutrons included in the neutron ray N and a layer 39B that mainly decelerates epithermal neutrons included in the neutron ray N.

The shield member 8 shields the generated neutron ray N and gamma rays or the like generated with the generation of the neutron ray N from being released to the outside. The shield member 8 is provided to surround the deceleration member 39. An upper portion and a lower portion of the shield member 8 extend upstream of the particle beam R beyond the deceleration member 39.

The collimator 20 shapes an irradiation field of the neutron ray N, and has an irradiation opening 20a through which the neutron ray N passes. The collimator 20 is, for example, a block-shaped member having the irradiation opening 20a at a center. The collimator 20 is attached to the irradiation section peripheral wall 115, which is a wall portion at a location where an inside of the treatment room 101 is irradiated with the neutron ray N.

The moving mechanism 110 is a so-called six-axis mechanism that moves the treatment bed 100 on which the patient 50 is placed in six axial directions. The moving mechanism 110 allows the treatment bed 100 to move horizontally and to rotate. In the present embodiment, the moving mechanism 110 supports the patient 50 in a state of being placed on the treatment bed 100, and moves the patient 50 together with the treatment bed 100.

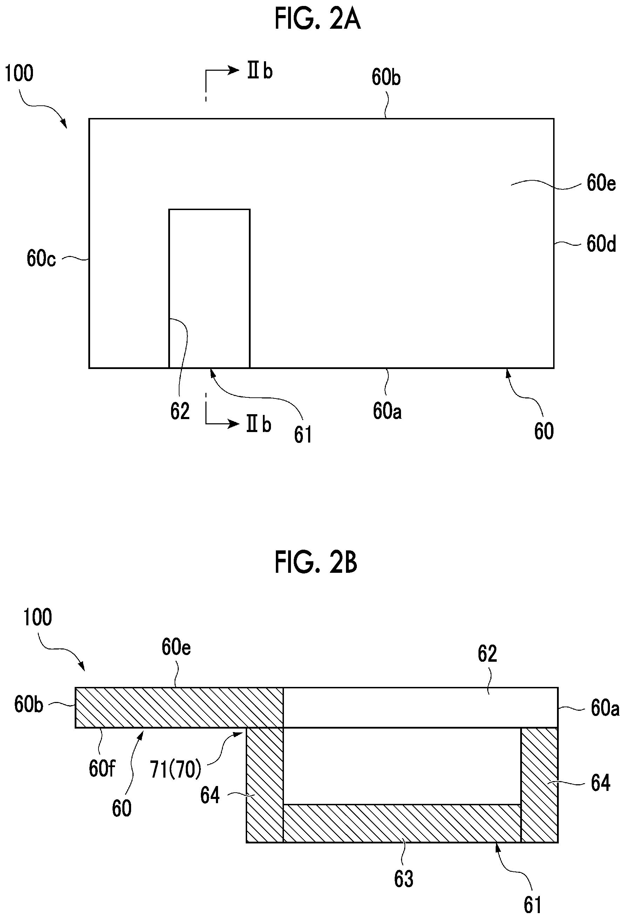

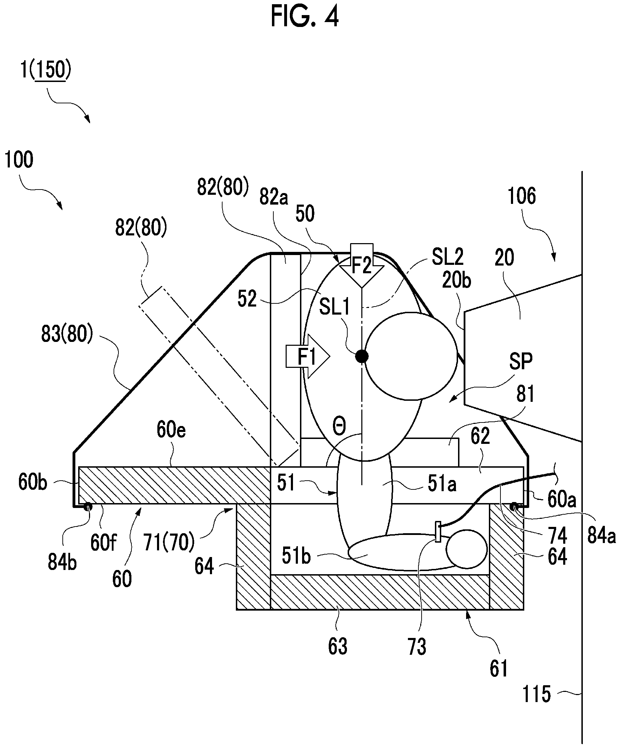

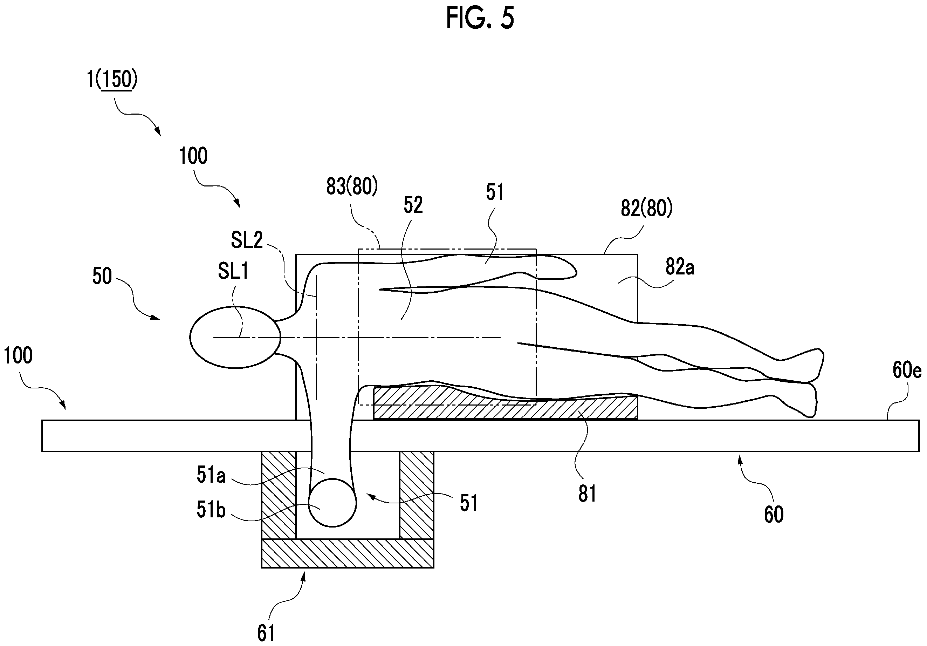

Next, a detailed configuration of the treatment bed 100 will be described with reference to Figs. 2A to 5. Fig. 2A is a schematic plan view of the treatment bed 100. Fig. 2B is a schematic sectional view taken along line IIb-IIb shown in Fig. 2A. Fig. 3 is a schematic plan view showing the treatment bed 100 in a state of being loaded with the patient 50. Fig. 4 is a schematic sectional view showing the treatment bed 100 in the state of being loaded with the patient 50. Fig. 5 is a schematic side view showing the treatment bed 100 in the state of being loaded with the patient 50.

As shown in Figs. 2A and 2B, the treatment bed 100 includes the placement section 60 and a support section 61. The placement section 60 is a member for placing the patient 50. The placement section 60 is a rectangular plate-shaped member. The placement section 60 has edge portions 60a and 60b on a long side and edge portions 60c and 60d on a short side. The placement section 60 has an upper surface 60e that is a placement surface on which the patient 50 is placed (see Fig. 3).

As shown in Fig. 2A, the placement section 60 includes an arm passing portion 62 through which an arm 51 of the placed patient 50 passes (see also Figs. 4 and 5). The arm passing portion 62 is formed of a notch or a through-hole that penetrates the placement section 60 in an up-down direction. In the present embodiment, the arm passing portion 62 is configured by a cutout portion extending from the edge portion 60a on the long side to the edge portion 60b on the long side. The arm passing portion 62 is formed at a position closer to the edge portion 60c on the short side. As shown in Figs. 3 and 4, the patient 50 placed on the upper surface 60e of the placement section 60 in a lateral position can extend the arm 51 on a placement section 60 side downward through the arm passing portion 62.

As shown in Fig. 2B, the support section 61 supports the arm 51 passing through the arm passing portion 62 (see Figs. 4 and 5). The support section 61 has a bottom wall portion 63 parallel to the placement section 60 at a position separated downward from the arm passing portion 62. In addition, the support section 61 has a side wall portion 64 extending upward from an edge portion of the bottom wall portion 63. As shown in Fig. 4, the patient 50 extends an upper arm 51a downward from the arm passing portion 62, bends an elbow, and places a forearm 51b on the bottom wall portion 63. In addition, the forearm 51b placed on the bottom wall portion 63 is protected by being surrounded by the side wall portion 64.

The support section 61 has a maintenance structure 70 for maintaining a relative positional relationship with the placement section 60. Here, maintaining the relative positional relationship means that a slight change in a relative position of the support section 61 with respect to the placement section 60 is allowed within a range in which a function as the support section 61 for supporting the arm 51 can be maintained, as well as in a state where the relative position of the support section 61 with respect to the placement section 60 is completely constant. In addition, the maintenance structure 70 allows not only complete synchronization between movement of the placement section 60 and movement of the support section 61, but also a slight deviation between the movements of the two.

In the present embodiment, the maintenance structure 70 is configured by a connecting portion 71 of the support section 61 to the placement section 60. The connecting portion 71 connects an upper end portion of the side wall portion 64 and a lower surface 60f of the placement section 60. Accordingly, the support section 61 is fixed to the placement section 60. Therefore, when the placement section 60 moves, the support section 61 also moves in synchronization with the movement. In addition, the relative position of the support section 61 with respect to the placement section 60 remains completely constant. The maintenance structure 70 may adopt a configuration other than the connecting portion 71 to the placement section 60. For example, the support section 61 may be separated from the placement section 60, and the support section 61 may be provided on a bogie that is movable on a floor surface. In this case, the connecting portion 71 is disposed below the arm passing portion 62 of the placement section 60.

Next, states of the patient 50 on the treatment bed 100 will be described with reference to Figs. 3 to 5. The particle beam treatment system 150 irradiates the patient 50 with the neutron ray N from the irradiation port 106 in a state where the patient 50 placed on the placement section 60 is fixed in the lateral position. The fixing here is to maintain a state of the lateral position such that the patient 50 does not maintain a posture by his/her own power during a treatment. A fixing force is not particularly limited, but it is preferable that the patient 50 can maintain a posture of the lateral position even when a force of about 10 N to 50 N is applied. The treatment bed 100 includes a support member 80 that supports the patient 50, and a pad member 81.

Here, the state of the lateral position of the patient 50 will be described. The lateral position is a state where the patient 50 lies sideways with one of right and left arms of the patient 50 downward. An imaginary reference line SL1 extending in a longitudinal direction is set with respect to a torso 52 of the patient 50. In addition, an imaginary reference line SL2 that is perpendicular to the reference line SL1 and extends in a direction in which both right and left shoulders widen is set. An angle θ of the reference line SL2 with respect to the upper surface 60e of the placement section 60 is defined when the patient 50 is set in the longitudinal direction (direction shown in Fig. 4). The lateral position applies not only in a case where the angle θ is 90° but also when the angle θ is within a range of 30° to 90° (rightward and leftward directions may be interchanged). In addition, even in a case where the patient 50 twists his/her body, when the angle θ in the vicinity of the shoulder is within the above range, the patient 50 is considered to be in the lateral position.

In the present embodiment, the patient 50 is disposed such that the reference line SL1 is substantially perpendicular to an irradiating direction of the neutron ray N and extends along the horizontal direction. Therefore, the treatment bed 100 is disposed such that the longitudinal direction of the placement section 60 is substantially perpendicular to the irradiating direction of the neutron ray N and extends along the horizontal direction. In addition, the patient 50 is disposed such that a front side of the patient 50 faces the irradiation section peripheral wall 115. Here, a head or a neck area of the patient 50 is disposed in front of the irradiation port 106.

The support member 80 is a member that supports the patient 50 placed on the placement section 60. In the present embodiment, the support member 80 includes a backrest member 82 and a belt-shaped member 83. The backrest member 82 is a member that stands upward from the upper surface 60e of the placement section 60. The backrest member 82 extends along the longitudinal direction of the placement section 60. The backrest member 82 supports a back of the torso 52 of the patient 50 on a support surface 82a. The support surface 82a may be made of a cushioning material. As shown in Fig. 4, in a case where the angle θ of the reference line SL2 of the patient 50 with respect to the placement section 60 is 90°, an angle of the backrest member 82 with respect to the placement section 60 is 90°. Accordingly, the backrest member 82 can support the posture of the lateral position of the patient 50 in a state of being in contact with the back of the patient 50. In a case where the angle θ of the patient 50 is inclined with respect to the placement section 60, the angle of the backrest member 82 with respect to the placement section 60 is inclined with respect to the placement section 60 so as to be the same as the angle θ of the patient 50 (see an imaginary line in Fig. 4).

The belt-shaped member 83 is a member that can be wound around the patient 50. The belt-shaped member 83 is an elongated member having a predetermined width and extending in the longitudinal direction. In addition, the belt-shaped member 83 can be freely deformed at each portion. The belt-shaped member 83 is made of a fiber material or a resin material. A belt is adopted as the belt-shaped member 83. In Figs. 3 and 5, the belt-shaped member 83 is shown by an imaginary line to show a state of the patient. In the present embodiment, the belt-shaped member 83 has a width dimension sufficient to cover substantially the entire torso 52 of the patient 50. In the present embodiment, one wide belt-shaped member 83 is used. However, a plurality of belt-shaped members 83 having a narrower width may be used.

As shown in Fig. 4, the belt-shaped member 83 is fixed in the vicinity of the edge portion 60a, wraps around upper ends of the patient 50 and the backrest member 82, and is fixed in the vicinity of the edge portion 60b. The belt-shaped member 83 wraps around the edge portions 60a and 60b up to a lower surface 60f side. The support member 80 has fulcrums 84a and 84b that hold end portions of the belt-shaped member 83 at a position of the lower surface 60f. In the present embodiment, the fulcrums 84a and 84b are disposed at positions lower than the upper surface 60e of the placement section 60. Accordingly, the belt-shaped member 83 draws a mountain shape, and supports the patient 50 with a supporting force F2 that presses the patient 50 downward against the placement section 60. In addition, the backrest member 82 applies a supporting force F1 to the torso 52 of the patient 50 in a direction perpendicular to the support surface 82a.

The pad member 81 is a member that is interposed in a space between the patient 50 in the state of the lateral position and the placement section 60. The pad member 81 is disposed at a position corresponding to the torso 52 of the patient 50 on the upper surface 60e of the placement section 60. When the patient 50 is in the posture of the lateral position on the placement section 60, one shoulder and arm 51 are located on a lower side. The pad member 81 fills a space formed between the shoulder and the arm 51, and the torso 52. The pad member 81 may be made of a cushioning material.

Next, a procedure when the patient 50 is treated using the treatment bed 100 will be described with reference to Figs. 3 and 4. As shown in Figs. 3 and 4, first, the patient 50 is placed on the upper surface 60e of the placement section 60 of the treatment bed 100 in the state of the lateral position. The patient 50 places the torso 52 on the pad member 81 on the placement section 60. In addition, the patient 50 leans his/her back against the backrest member 82. The patient 50 passes the arm 51 on the lower side through the arm passing portion 62 and extends the arm 51 downward of the placement section 60. In addition, the elbow is bent, and the forearm 51b is placed on the bottom wall portion 63 of the support section 61. Next, an IV drip 73 is inserted into the forearm 51b supported by the support section 61. A tube 74 of the IV drip 73 is drawn from the arm passing portion 62. The treatment bed 100 fixes the patient 50 with the inserted IV drip 73 by means of the belt-shaped member 83.

In this way, the patient 50 is fixed by the support member 80 in a state of being in the lateral position. Accordingly, the head and neck area of the patient 50 can be disposed in front of a front surface of the collimator 20 of the irradiation port 106, and the posture can be maintained. The neutron capture therapy apparatus 1 irradiates an affected part of the patient 50 in a state of being fixed in the lateral position with the neutron ray N by emitting the neutron ray N from the collimator 20.

Next, operation and effects of the particle beam treatment system 150 according to the present embodiment will be described.

For example, in a proton beam treatment apparatus, since a charged particle beam can be bent, it is easy to provide a mechanism for moving an irradiation opening with a gantry. Therefore, the irradiation opening can be disposed at a position corresponding to an affected part to be treated of a patient lying on his/her back on a treatment bed, by the gantry. On the other hand, in the case of the neutron capture therapy apparatus 1, the neutron ray N cannot be bent, and thus it is difficult to provide a moving mechanism such as a gantry. Therefore, a structure may be adopted in which the neutron ray N is emitted from the collimator 20 of the irradiation port 106 in the horizontal direction. In an apparatus that performs emission in the horizontal direction from the irradiation port 106 as described above, in a case where a treatment site near a clavicle of the patient 50 is irradiated with the neutron ray N, it is conceivable to irradiate the treatment site obliquely in consideration of a burden on the patient 50. In a case of irradiating a cervical region (neck), an irradiating direction is changed depending on a position of the affected part in the neck. In such a case, it may be better to irradiate the treatment site obliquely even without considering the burden on the patient 50. However, in the case of the neutron capture therapy apparatus 1, since high-dose irradiation is performed, there is a possibility that a dose shortage may occur in a case where the irradiation is performed obliquely. The treatment site can be brought closest to an emission port of the neutron ray N in a case where the neutron ray N is irradiated from directly beside the patient 50 (at 90° with respect to the reference line SL1 of the torso 52). Therefore, in the present embodiment, the patient 50 is placed on the placement section 60 such that the reference line SL1 is substantially perpendicular to the irradiating direction of the neutron ray N (see Figs. 3 and 4).

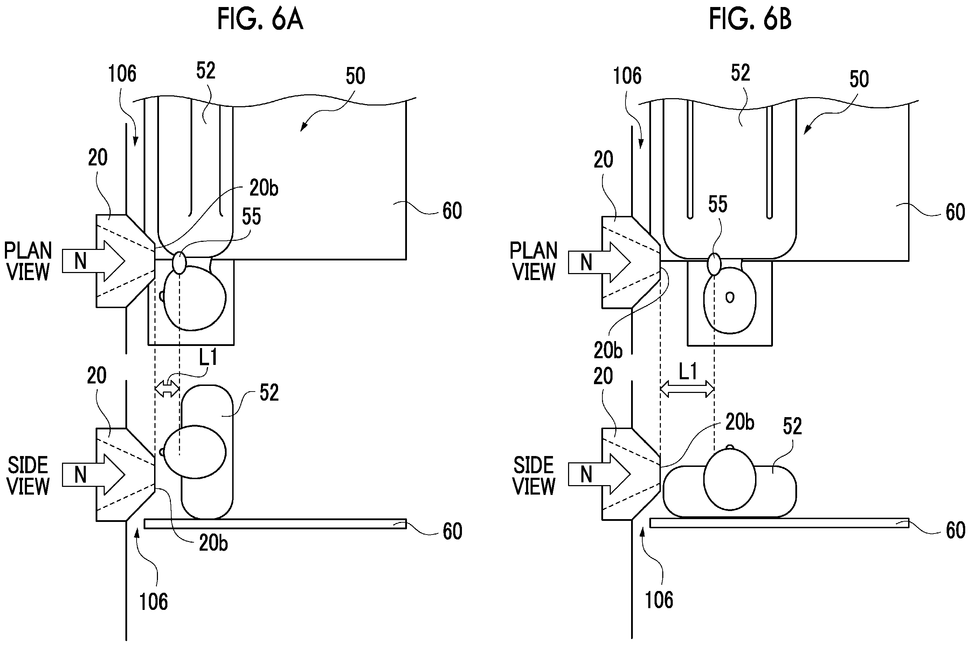

Here, a distance between a treatment site 55 of the patient 50 and an emission port 20b of the collimator 20 will be described with reference to Figs. 6A and 6B. Fig. 6A is a plan view and a side view showing a state where the patient 50 is placed on the placement section 60 in the lateral position. Fig. 6B is a plan view and a side view showing a state where the patient 50 is placed on the placement section 60 in a supine position. As shown in Fig. 6B, in a case where the treatment site 55 is present near the clavicle, even if the emission port 20b is brought close to the treatment site 55, the collimator 20 and the vicinity of the shoulder of the patient 50 interfere with each other, and thus a distance L1 between the emission port 20b and the treatment site 55 increases. Such a problem is particularly likely to occur when a portion smaller than the torso 52 such as the neck or chin as well as the clavicle is irradiated with the neutron ray N in a direction intersecting the reference line SL1 of the patient 50.

In response to such a problem, the particle beam treatment system 150 according to the present embodiment includes the placement section 60 for placing the patient 50 to be irradiated with the particle beam. Therefore, the irradiation port 106 can irradiate the treatment site 55 of the patient 50 placed on the placement section 60 with the particle beam. With regard to this, the particle beam treatment system 150 irradiates the patient 50 with the particle beam from the irradiation port 106 in a state where the patient 50 placed on the placement section 60 is fixed in the lateral position. For example, as shown in Fig. 6A, when the patient 50 is in the lateral position, interference between the collimator 20 and the vicinity of the shoulder can be suppressed. Therefore, the distance L1 between the emission port 20b and the treatment site 55 can be reduced. In this way, by placing the patient 50 in the lateral position according to the position of the treatment site 55 of the patient 50, the treatment site 55 can be disposed near the emission port 20b of the irradiation port 106. Furthermore, by fixing the posture of the lateral position of the patient in a state where the treatment site 55 is disposed near the emission port 20b, it is possible to suppress movement of the treatment site 55 during the treatment. Accordingly, the treatment can be reliably performed by performing accurate irradiation with the particle beam.

The particle beam treatment system 150 may further include the support member 80 capable of supporting the patient 50 placed on the placement section 60 from at least one side with respect to the torso 52. The patient 50 can be easily fixed in the lateral position by using the support member 80. In addition, by supporting the patient 50 from at least one side with respect to the torso 52, the movement of the patient 50 is suppressed, and thus it is easy for the patient to maintain a comfortable posture.

The support member 80 may include the belt-shaped member 83 that can be wound around the patient 50. The patient 50 can be fixed according to a physique or posture of the patient 50 by using the belt-shaped member 83.

The particle beam treatment system 150 may further include the pad member 81 interposed in a space between the patient 50 in the state of the lateral position and the placement section 60. Accordingly, a space between the arm and the shoulder of the patient 50 in the state of the lateral position, the torso 52, and the placement section 60 can be filled with the pad member 81. Therefore, even in a case where the patient 50 is in the lateral position with one arm and shoulder placed on the lower side, the movement of the patient 50 is suppressed, and it is easy for the patient 50 to maintain a comfortable posture.

The particle beam treatment system 150 may include the neutron capture therapy apparatus 1. In the case of the neutron capture therapy apparatus 1, since it is necessary to perform high-dose irradiation, it is highly necessary to bring the treatment site 55 of the patient 50 close to the emission port 20b of the irradiation port 106. Therefore, compared to a proton beam treatment or the like, the patient 50 is more likely to be in a posture that is burdensome for the patient 50, such as a lateral position. Furthermore, since an irradiation time is long, a time for which the burden is applied to the patient 50 increases. Therefore, by adopting the structure of the present embodiment for the neutron capture therapy apparatus 1, it is possible to suppress the burden on the patient 50 and to perform accurate irradiation with the neutron ray N.

The support member 80 for a particle beam treatment according to the present embodiment is the support member 80 for a particle beam treatment that supports the patient 50 to be irradiated with the particle beam, and is capable of fixing the patient 50 placed on the placement section 60 in the state of the lateral position.

The support member 80 for a particle beam treatment according to the present embodiment can fix the patient 50 placed on the placement section 60 in the state of the lateral position. Therefore, by placing the patient 50 in the lateral position using the support member 80 according to the position of the treatment site 55 of the patient 50, the treatment site 55 can be disposed near the emission port 20b. Furthermore, a state where the posture of the lateral position of the patient 50 is fixed by the support member 80 can be maintained in a state where the treatment site 55 is disposed near the emission port 20b, so that it is possible to suppress the movement of the treatment site 55 during the treatment. Accordingly, the treatment can be reliably performed by performing accurate irradiation with the particle beam.

The present invention is not limited to the above-described embodiment.

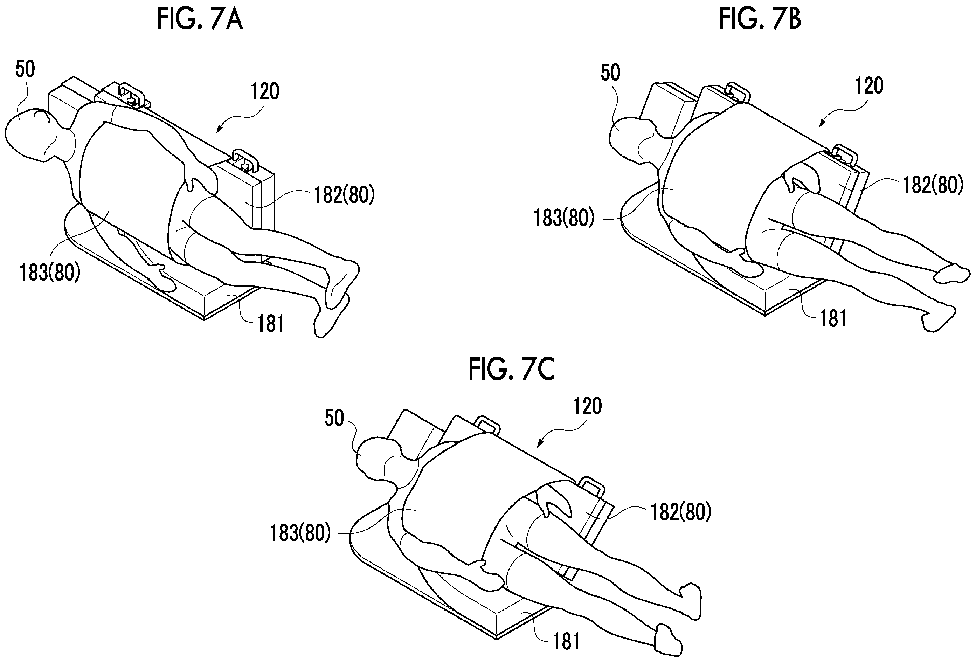

For example, a support unit 120 as shown in Figs. 7A to 7C may be adopted. The support unit 120 is unitized by a backrest member 182 (first member), a belt-shaped member 183 (second member), and a pad member 181. The belt-shaped member 183 is attached to the backrest member 182. In the support unit 120, an angle between the backrest member 182 and the belt-shaped member 183, and the pad member 181 is set according to the angle θ (see Fig. 4) with respect to the placement section 60 of the patient 50. Fig. 7A is a perspective view showing the support unit 120 in which the backrest member 182 is set at 90° with respect to the placement section 60. Fig. 7B is a perspective view showing the support unit 120 in which the backrest member 182 is set at 60° with respect to the placement section 60. Fig. 7C is a perspective view showing the support unit 120 in which the backrest member 182 is set at 45° with respect to the placement section 60.

In the support unit 120, the backrest member 182 and the belt-shaped member 183 may be capable of adjusting the angle with respect to the pad member 181 (that is, the placement section 60). That is, the angle of the backrest member 182 and the belt-shaped member 183 can be changed according to the angle θ of the patient 50 with respect to the placement section 60. Alternatively, the angle of the backrest member 182 and the belt-shaped member 183 with respect to the pad member 181 (that is, the placement section 60) may be fixed and non-adjustable. In this case, a plurality of support units 120 may be prepared according to the angle θ of the patient 50 with respect to the placement section 60.

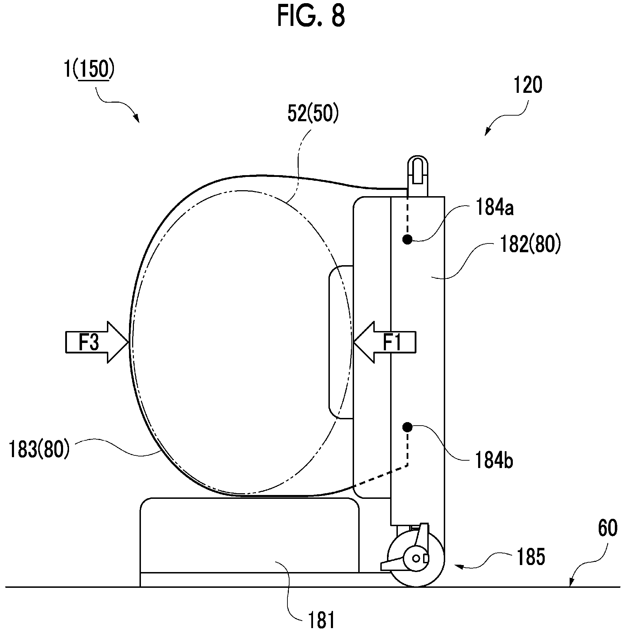

A configuration of the support unit 120 will be described in detail with reference to Fig. 8. Fig. 8 is a view of the support unit 120 as viewed in the longitudinal direction. As shown in Fig. 8, one end portion of the pad member 181 and a lower end portion of the backrest member 182 are connected to each other via a connecting portion 185. In a case where the angle of the backrest member 182 is adjustable, a hinge mechanism that rotatably supports the backrest member 182 relative to the pad member 181 and locks the backrest member 182 at a desired angle is provided in the connecting portion 185.

One end portion of the belt-shaped member 183 is held on a lower end side of the backrest member 182, and the other end portion thereof is held on an upper end side of the backrest member 182. One end portion of the belt-shaped member 183 wraps around a back side from the lower end side of the backrest member 182 and is held at a fulcrum 184b. The other end portion of the belt-shaped member 183 wraps around the back side from the upper end side of the backrest member 182 and is held at a fulcrum 184a. Both of the fulcrums 184a and 184b are disposed at positions higher than the placement section 60.

The support member 80 of the support unit 120 can support the patient 50 placed on the placement section 60 from both sides with respect to the torso 52. As shown in Fig. 8, the belt-shaped member 183 is wound around the torso 52 of the patient 50 placed on the pad member 181 in a state of a lateral position (see Fig. 7A). Since the belt-shaped member 183 is held at the fulcrums 184a and 184b on the upper end side and the lower end side of the backrest member 182, a supporting force F3 is applied by tightening the belt-shaped member 183 so as to press the backrest member 182 against the patient 50. As a repulsive force, a supporting force F1 is applied to the patient 50 from the backrest member 182. In this way, by supporting the patient 50 from both sides with respect to the torso 52, the movement of the patient 50 is suppressed, and thus it is easy for the patient 50 to maintain a comfortable posture.

The state of being supported from both sides with respect to the torso 52 is a state where the support member 80 can apply a supporting force to the torso 52 of the patient 50 in at least two directions, and the supporting forces act in opposite directions across the torso 52. The supporting forces in the two directions act in a direction perpendicular to the longitudinal direction of the placement section 60 and extending along the upper surface of the placement section 60. For example, in a form of Fig. 4, the supporting force F2 of the belt-shaped member 83 is directed downward, and does not act in the opposite direction to the supporting force F1 of the backrest member 82. Therefore, the form does not correspond to the state of being supported from both sides with respect to the torso 52.

As described above, in forms shown in Figs. 7A to 8, the support member 80 has the fulcrum 184a that holds one end portion of the belt-shaped member 83, and the fulcrum 184a is disposed at a position higher than the placement section 60. For example, in a case where the fulcrums of both end portions of the belt-shaped member 83 are located at positions lower than the placement section (for example, see Fig. 4), the belt-shaped member 83 applies a supporting force to press the patient toward a placement section side. Contrary to this, in a case where the fulcrum 184a is disposed at a position higher than the placement section 60, it is possible to apply a supporting force in a direction other than the direction in which the placement section 60 is pressed. Accordingly, it is easy to apply supporting forces to the patient 50 from both sides with respect to the torso 52 of the patient 50.

The support member 80 has the backrest member 182 (first member) and the belt-shaped member 183 (second member) that support the patient 50 placed on the placement section 60 from both sides with respect to the torso 52. By using the support member 80 unitized by the backrest member 182 (first member) and the belt-shaped member 183 (second member), it is possible to easily position and fix the patient 50.

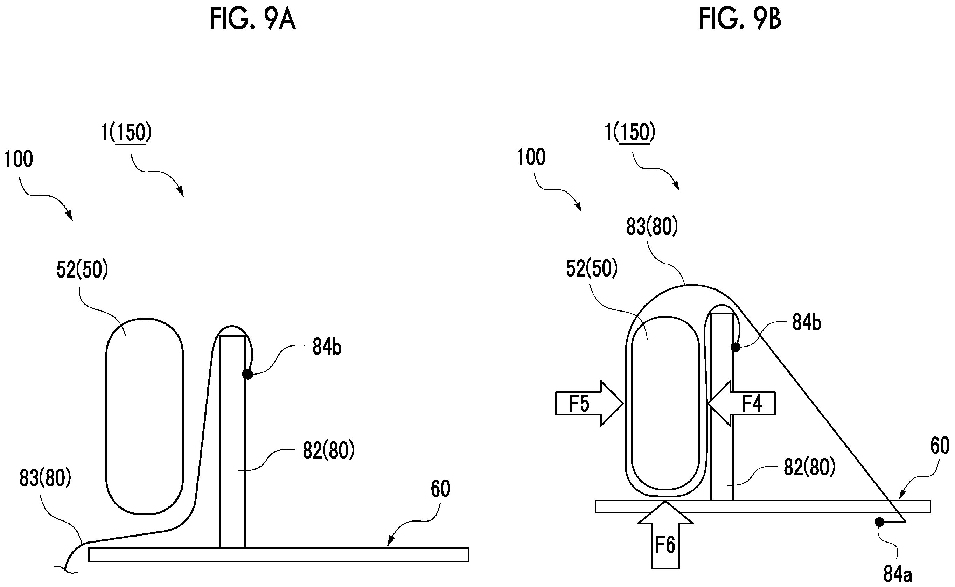

Structures as shown in Figs. 9A to 10 may be adopted. In a form shown in Figs. 9A and 9B, one end portion of the belt-shaped member 83 is held at the fulcrum 84b on an upper end side of the backrest member 82. As shown in Fig. 9A, the belt-shaped member 83 is wrapped around an upper end of the backrest member 82 from the fulcrum 84b and is lowered downward to be disposed on the placement section 60. In this state, the patient 50 is disposed on the belt-shaped member 83 disposed on the placement section 60.

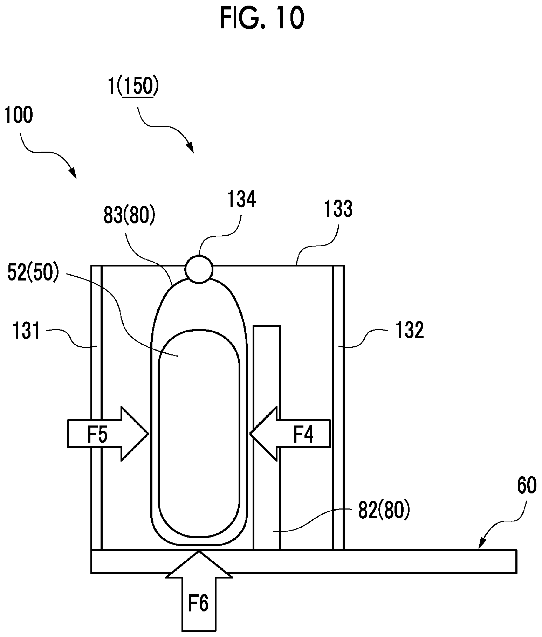

Next, as shown in Fig. 9B, the belt-shaped member 83 is wound around the torso 52 of the patient 50, and the other end portion of the belt-shaped member is wrapped around the upper end of the backrest member 82 and pulled toward a lower surface side of the placement section 60. Then, the other end portion of the belt-shaped member 83 is held at the fulcrum 84a on the lower surface of the placement section 60 in a state where a tension is applied so that the patient 50 is tightened. Here, the belt-shaped member 83 is wrapped around a lower side of the torso 52 from a side surface of the torso 52 on one side in the horizontal direction, and is wound to be wrapped around a side surface of the torso 52 on the other side in the horizontal direction. Accordingly, supporting forces F4 and F5 in opposite directions are applied to the torso 52 of the patient 50 from both sides in the horizontal direction, and a supporting force F6 directed upward from the lower side is applied. In Figs. 9A and 9B, a gap is provided between the belt-shaped member 83 and other members and the patient 50 in order to facilitate understanding of the state of the belt-shaped member 83. However, in reality, the belt-shaped member 83 is in contact with other members and the patient 50 without any gaps. The same applies to Fig. 10 described later.

As shown in Fig. 10, the patient 50 may be hung by the belt-shaped member 83. The placement section 60 is provided with pillar members 131 and 132 on both sides of the backrest member 82, and a wire 133 is provided between the upper ends of the pillar members 131 and 132. The wire 133 is provided with a fulcrum 134 that holds both end portions of the belt-shaped member 83. In a state where the belt-shaped member 83 is wound around the torso 52 of the patient 50, the belt-shaped member 83 together with the patient 50 is hung at the fulcrum 134. In this case, the torso 52 of the patient 50 is tightened by the belt-shaped member 83 due to an action of gravity. Accordingly, the supporting forces F4 and F5 in opposite directions are applied to the torso 52 of the patient 50 from both sides in the horizontal direction, and the supporting force F6 directed upward from the lower side is applied.

As described above, the support member 80 shown in Figs. 9A to 10 can apply the supporting forces to the torso 52 from the lower side and from both sides in the horizontal direction by winding the belt-shaped member 83 around the torso 52 of the patient 50 placed on the placement section 60. In this case, by applying the supporting forces in multiple directions according to the physique and posture of the patient 50 by using the belt-shaped member 83, the movement of the patient 50 is suppressed, and thus it is easy for the patient to maintain a comfortable posture. In the form shown in Fig. 4, since the supporting force F2 is applied to press the patient 50 against the placement section 60, there may be a burden on the shoulder or the like of the patient 50. Contrary to this, in Figs. 9A to 10, since the supporting force F6 directed upward from the lower side is applied, the burden on the shoulder or the like of the patient 50 can be reduced. In addition, in the form shown in Fig. 4, a large gap SP is likely to be formed between the torso of the patient 50 and the belt-shaped member 83, and there is a possibility that a fixing force decreases. On the other hand, in the forms of Figs. 9A to 10 (and further Fig. 8), the formation of such a gap SP can be suppressed, and thus, the decrease in the fixing force can be suppressed.

In the forms shown in Figs. 9A to 10, the support member 80 has the fulcrums 84b and 134 for holding one end portion of the belt-shaped member 83, and the fulcrums 84b and 134 are disposed at positions higher than the placement section 60. In a case where the fulcrums 84b and 134 are disposed at positions higher than the placement section 60, it is possible to apply a supporting force in a direction other than the direction in which the placement section 60 is pressed as shown in Fig. 4. Accordingly, it is easy to apply supporting forces to the patient 50 from both sides with respect to the torso 52 of the patient 50.

In the above-described embodiment and modification examples, the support member for the neutron capture therapy apparatus has been exemplified. However, a treatment apparatus included in the particle beam treatment system is not particularly limited, and a wide range of treatment apparatuses using a particle beam such as a proton beam treatment apparatus, a heavy particle beam apparatus, or an X-ray device can be applied.

### Brief Description of the Reference Symbols

1 neutron capture therapy apparatus
50 patient
60 placement section
80 support member
81 pad member
83 belt-shaped member
182 backrest member (first member)
183 belt-shaped member (second member)
106 irradiation port (irradiation section)

## Claims

1. A particle beam treatment system (150) comprising:
a placement section (60) for placing a patient (50) to be irradiated with a particle beam; and
an irradiation section (106) that irradiates the patient (50) placed on the placement section (60) with the particle beam, wherein the irradiation section (106) irradiates the patient (50) with the particle beam in a state where the patient (50) placed on the placement section (60) is fixed in a lateral position.

2. The particle beam treatment system (150) according to claim 1, further comprising:
a support member (80) that is capable of supporting the patient (50) placed on the placement section (60) from at least one side with respect to a torso (52) of the patient (50).

3. The particle beam treatment system (150) according to claim 2,
wherein the support member (80) is capable of supporting the patient (50) placed on the placement section (60) from both sides with respect to the torso (52).

4. The particle beam treatment system (150) according to claim 2,
wherein the support member (80) includes a belt-shaped member (83, 183) that is windable around the patient (50).

5. The particle beam treatment system (150) according to claim 4,
wherein the support member (80) includes a fulcrum (84a, 84b, 134, 184a, 184b) that holds one end portion of the belt-shaped member (83, 183), and
the fulcrum (84a, 84b, 134, 184a, 184b) is disposed at a position higher than the placement section (60).

6. The particle beam treatment system (150) according to claim 4,
wherein the support member (80) applies a supporting force (F1, F2, F3, F4, F5, F6) to the torso (52) from below and from both sides in a horizontal direction by winding the belt-shaped member (83, 183) around the torso (52) of the patient (50) placed on the placement section (60).

7. The particle beam treatment system (150) according to claim 3,
wherein the support member (80) includes a first member that supports the patient (50) placed on the placement section (60) from both sides with respect to the torso (52), and a second member.

8. The particle beam treatment system (150) according to claim 1, further comprising:
a pad member (81, 181) interposed in a space between the patient (50) in a state of the lateral position and the placement section (60).

9. The particle beam treatment system (150) according to claim 1, further comprising:
a neutron capture therapy apparatus (1).

10. A support member (80) for a particle beam treatment that supports a patient (50) to be irradiated with a particle beam,
wherein the support member (80) for a particle beam treatment is capable of fixing the patient (50) placed on a placement section (60) in a state of a lateral position.
